# EUROPEAN PATENT SPECIFICATION

(11) **EP 3 019 162 B1**
(45) Date of publication and mention of the grant of the patent: **19.05.2021**
(21) Application number: 14737227.0
(22) Date of filing: 08.07.2014
(51) Int. Cl.: A61K 31/122, A61K 31/343, A61K 36/537, A61P 25/08

(54) **DAN SHEN EXTRACTS AND COMPOUNDS**
DANSHEN-EXTRAKTE UND VERBINDUNGEN
EXTRAITS ET COMPOSÉS DE SAUGE ROUGE (DAN SHEN)

(30) Priority: 08.07.2013 GB 201312205
(43) Date of publication of application: 18.05.2016
(73) Proprietor: De Witte, Peter, B-3010 Kessel-Lo (BE); Esguerra, Camila, B-1315 Gilmes (BE); Crawford, Alexander, B-1315 Gilmes (BE); Buenafe, Olivia, B-3000 Leuven (BE); Luyten, Walter, B-3060 Bertem (BE); De Borggraeve, Wim, B-3000 Leuven (BE); Tsinghua University, Haidian District, Beijing 100084 (CN)
(72) Inventor: LUO, Guoan, Beijing 100084 (CN); LIANG, Qionglin, Beijing 100084 (CN); WANG, Yiming, Beijing 100084 (CN); LIU, Qingfei, Beijing 100084 (CN); DE WITTE, Peter, B-3010 Kessel-LO (BE); ESGUERRA, Camila, B-1315 Gilmes (BE); CRAWFORD, Alexander, B-1315 Gilmes (BE); BUENAFE, Olivia, B-3000 Leuven (BE); LUYTEN, Walter, B-3060 Bertem (BE); DE BORGGRAEVE, Wim, B-3000 Leuven (BE)
(74) Representative: Grund, Martin
(86) International application number: PCT/EP2014/064513
(87) International publication number: WO 2015/004093

(56) References cited:
- WO-A1-02/12218
- WO-A1-96/35441
- WO-A2-2007/084419
- WANG W M ET AL: "REPAIRED ABNORMAL PERFUSION FOCI IN CHILDREN WITH EPILEPSY THROUGH TRADITIONAL CHINESE MEDICINE COMPOUND DANSHEN DRIPPING PILLS ASSISTED WITH VALPROIC ACID (110 CASE): TO EVALUATE IN A NEW TREATMENT THROUGH INTERICTCAL SPECT AND LONG-TERM V-EEG AND IMAGING", EPILEPSIA, RAVEN PRESS LTD, NEW YORK, US, vol. 54, no. Suppl.3, 1 June 2013 (2013-06-01), pages 61-62, XP009180235, ISSN: 0013-9580
- DATABASE WPI Week 201267 Thomson Scientific, London, GB; AN 2012-L72554 XP002729960, & CN 102 579 989 A (SHANDONG RENHETANG PHARM CO LTD) 18 July 2012 (2012-07-18)
- DATABASE WPI Week 200676 Thomson Scientific, London, GB; AN 2006-730692 XP002729961, & CN 1 785 409 A (XIAN RIANHE PHARM CO LTD) 14 June 2006 (2006-06-14)
- SUCHER ET AL: "Insights from molecular investigations of traditional Chinese herbal stroke medicines: Implications for neuroprotective epilepsy therapy", EPILEPSY AND BEHAVIOR, ACADEMIC PRESS, SAN DIEGO, CA, US, vol. 8, no. 2, 1 March 2006 (2006-03-01), pages 350-362, XP024941381, ISSN: 1525-5050, DOI: 10.1016/J.YEBEH.2005.11.015 [retrieved on 2006-03-01]
- JIA YONGLIANG ET AL: "Is danshen (Salvia miltiorrhiza) dripping pill more effective than isosorbide dinitrate in treating angina pectoris? A systematic review of randomized controlled trials", INTERNATIONAL JOURNAL OF CARDIOLOGY, vol. 157, no. 3, 14 June 2012 (2012-06-14) , pages 330-340, XP028921028, ISSN: 0167-5273, DOI: 10.1016/J.IJCARD.2010.12.073
- LIMIN ZHOU ET AL: "DANSHEN: AN OVERVIEW OF ITS CHEMISTRY, PHARMACOLOGY, PHARMCOKINETICS, AND CLINICAL USE", JOURNAL OF CLINICAL PHARMACOLOGY, LIPPINCOTT CO, HAGERSTOWN, MD, US, vol. 45, no. 12, 1 January 2005 (2005-01-01), pages 1345-1359, XP009069227, ISSN: 0091-2700, DOI: 10.1177/0091270005282630
- LI XIAO-YING ET AL: "Recommendations on the Clinical Use of Compound Danshen Dripping Pills", CHINESE MEDICAL JOURNAL (ENGLISH EDITION), vol. 130, no. 8, 20 April 2017 (2017-04-20), pages 972-978,
- OLIVIA ERIN BUENAFE ET AL: "Tanshinone IIA Exhibits Anticonvulsant Activity in Zebrafish and Mouse Seizure Models", ACS CHEMICAL NEUROSCIENCE, vol. 4, no. 11, 6 September 2013 (2013-09-06), pages 1479-1487, XP055531728, US ISSN: 1948-7193, DOI: 10.1021/cn400140e
- JIA CHEN ET AL: "Protective effect of compound Danshen (&ITSalvia miltiorrhiza&IT) dripping pills alone and in combination with carbamazepine on kainic acid-induced temporal lobe epilepsy and cognitive impairment in rats", PHARMACEUTICAL BIOLOGY, vol. 56, no. 1, 21 March 2018 (2018-03-21) , pages 217-224,

## Description

### FIELD OF INVENTION

The present disclosure relates to the anticonvulsant activity of extracts and compounds derived from *Salvia miltiorrhiza,* and their use in the treatment of epilepsy and neurological disorders characterized by seizures.

### BACKGROUND

Epilepsy affects approximately 60 million persons worldwide, with 30% of its population suffering from pharmacoresistant seizures. The economics of maintaining a cost-effective regimen hinges on their long-term efficacy and the probability of patients developing treatment-resistant seizures or possible side-effects such as sedation to Steven-Johnson syndrome [1]. To address such issues, there is an ongoing hunt for new AEDs with novel mechanisms of action with minimal or no side-effects.

There is a resurgence of interest in exploring plant, microbial and marine resources used in traditional herbal medicine for potential drug leads. According to the World Health Organization (WHO), 70% - 80% of populations in developing and developed countries have used or depended on such therapies, which became the impetus for efforts in bioprospecting and drug development [2]. Despite prevalent scepticism, as many as 25% of pharmaceutical drugs in the market are plant-based and were discovered through investigations on traditional or folk medicine practised by different cultures [1,2]. The rationale behind this approach in drug discovery is that small molecules isolated or derived from natural resources offer a more diverse set of structures compared to compounds synthesized through combinatorial techniques [2].

Each plant or marine extract can be treated as a potential library of hits, which can be screened by an appropriate medium- to high-throughput in vivo model such as zebrafish (*Danio rerio),* a freshwater teleost of the Cyprinidae family [3,4]. In screening for potential hits and leads for AED development, we have previously described the use of larval zebrafish as a platform for pinpointing AED-like activity of small molecules isolated from plant sources [3,4]. In a study conducted by Baraban et al., 7-dpf zebrafish larvae display seizure-like behavior (e.g., "whirlpool" swimming, loss of posture) upon exposure to a chemoconvulsant like pentylenetetrazole (PTZ) [5,6]; this physiological response to PTZ is found to be comparable to rodent models, with similar outcomes to treatment with known antiepileptic drugs [6,7,8]. This similarity, as well as other factors such as ease of propagation, sample introduction and the availability of automated scoring or tracking schemes, positions zebrafish as a robust model for pre-screening extracts (and eventually isolated compounds) from various ethnopharmacopeia, such as the *materia medica* of traditional Chinese medicine (TCM).

TCM is a branch of alternative therapy established more than 2500 years ago, with its own system of standards and diagnostic logic. Diagnoses are primarily syndrome-based-syndrome as the concrete manifestation of a fundamental pathological process affected by incidental aspects such as the patient's physical and psychological constitution and social environment [9]. For example, TCM describes an affliction called "internal wind syndrome" or "wind stroke", which is actually two diseases or disorders in Western medicine-strokes and seizures-as there is no direct TCM language equivalent for both [9].

Thus, there remains a need in the art for identifying and characterizing compounds and/or classes of compounds which may be used to treat different aspects of epilepsy. A random sampling of forty plants used to treat such condition in TCM therapy was initially screened by our research group; one plant was found positive in our screen-Chinese red sage or dan shen.

Dan shen *(Salvia miltiorrhiza,* Bunge) is a plant from TCM's *materia medica* that is often used in cardio- and cerebrovascular disorders [2,10], It is commonly prescribed by TCM practitioners to stroke patients, and is included in a TCM drug formulation *ding xian wan* to control epileptic seizures [9,11]. The active compounds documented in dan shen belong mostly to a specific diterpenoid class referred to as tanshinones, or are salvianolic acid derivatives. The former are isolated mainly via lipophilic extraction of the dried root powder and are also responsible for the characteristic red color of the herb (*dan shen* literally means "red root"). Both groups of compounds have different pharmacological activities. For example, tanshinone I inhibits arachidonic acid metabolism via phospholipase A2 [27,28], and salvianolic acids inhibit platelet aggregation and thrombosis [27,29]. Dan shen's predominant bioactive constituent, tanshinone IIA, has been a focus of research for the past decade in the field of cardiovascular and cerebral ischemia. The reported biological activities of tanshinone IIA are varied-anti-atherosclerotic [27,30], cardioprotective [9,30], and neuroprotective [31,32]. Because of the extensive preclinical and clinical studies on its cardioprotective and anti-atherosclerotic properties, tanshinone IIA and its more water-soluble derivative sodium tanshinone IIA sulfonate are used in China as prescription treatments for angina pectoris and stroke [30,32]. The neuroprotective effects of dan shen extracts on cerebral ischemia and Alzheimer's disease models have been elucidated, but scant data are available for its ascribed anticonvulsive effects, making it a potential library of small molecules to be screened in larval zebrafish for antiepileptic activity.

In an early attempt to analyze the effect of dan shen in the treatment of epilepsy, traditional Chinese medicine Compound Danshen Dripping Pills were combined with Magnesium valproate sustained-release tablets [33].

Further, tanshinones from S. *miltiorrhiza* were discovered to have *N*-methyl-D-aspartate receptor (NMDAR) blocking activity representing a potential new compound for development of therapy of NMDAR related disorders [34].

Described herein are compounds from dan shen for use as anticonvulsant agents to reduce seizures and symptoms of epilepsy.

### SUMMARY OF INVENTION

The invention is defined by the appended claims

One aspect of the present disclosure relates to an acetone extract of Salvia militorrhizhae for use as an anticonvulsant in treating disorders of the central nervous system. In some embodiments, the acetone extract comprises tanshinones. For example, the acetone extract may comprise dihydrotanshinone I, cryptotanshinone, tanshinone IIA, and miltirone. An exemplary extract may comprise tanshinone I. In certain aspects, the disorder of the central nervous system is characterized by seizures. In some embodiments, the disorder of the central nervous system is epilepsy.

A further aspect of the present disclosure relates to a mixture of tanshiones for use an anticonvulsant in treating disorders of the central nervous system. In some embodiments, the mixture comprises at least two of dihydrotanshinone I, cryptotanshinone, tanshinone IIA, and miltirone. In certain embodiments, the mixture comprises tanshinone IIA and at least one of dihydrotanshinone I, cryptotanshinone, and miltirone. An exemplary mixture may comprise tanshinone I, for example, tanshinone IIA and tanshinone I. The mixture of tanshinones may be used as an anticonvulsant for disorders of the central nervous system that are characterized by seizures. For example, the disorder of the central nervous system may be epilepsy.

Another aspect of the present disclosure relates to tanshinone IIA for use as an anticonvulsant in treating disorders of the central nervous system. In some aspects, the disorder of the central nervous system is characterized by seizures. In certain embodiments, the disorder of the central nervous system is epilepsy.

### BRIEF DESCRIPTION OF DRAWINGS

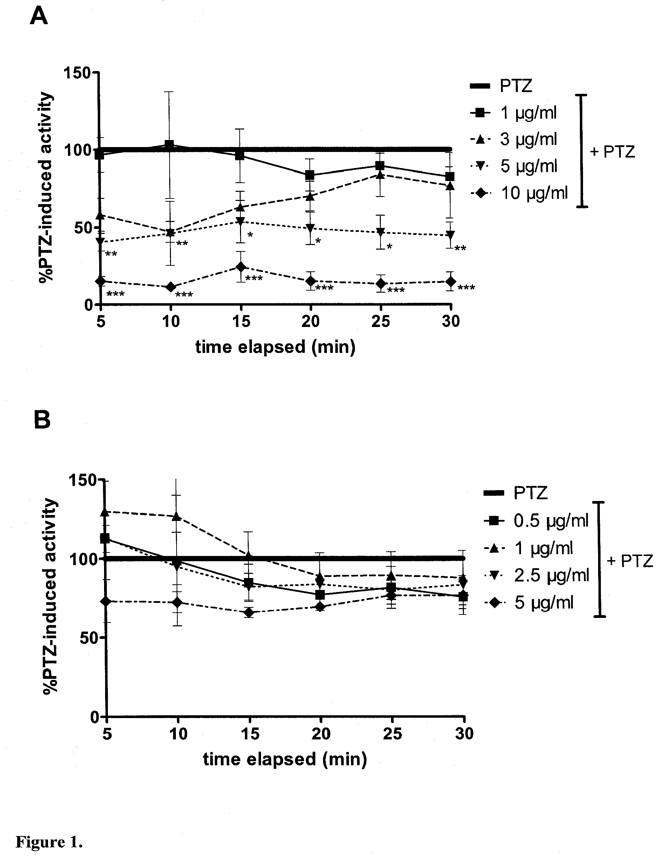

Figure 1 shows PTZ-induced activity curve of 7-dpf zebrafish larvae after pre-treatment with different concentrations of dan shen crude extract. Results were normalized against vehicle-only controls (set at 100%). Exposure time to extract at 1 hour (A) and 18 hours (B). Analysis was done by two-way ANOVA, with P values < 0.05 (*), < 0.01 (**) and < 0.001 (***) indicated per time period.

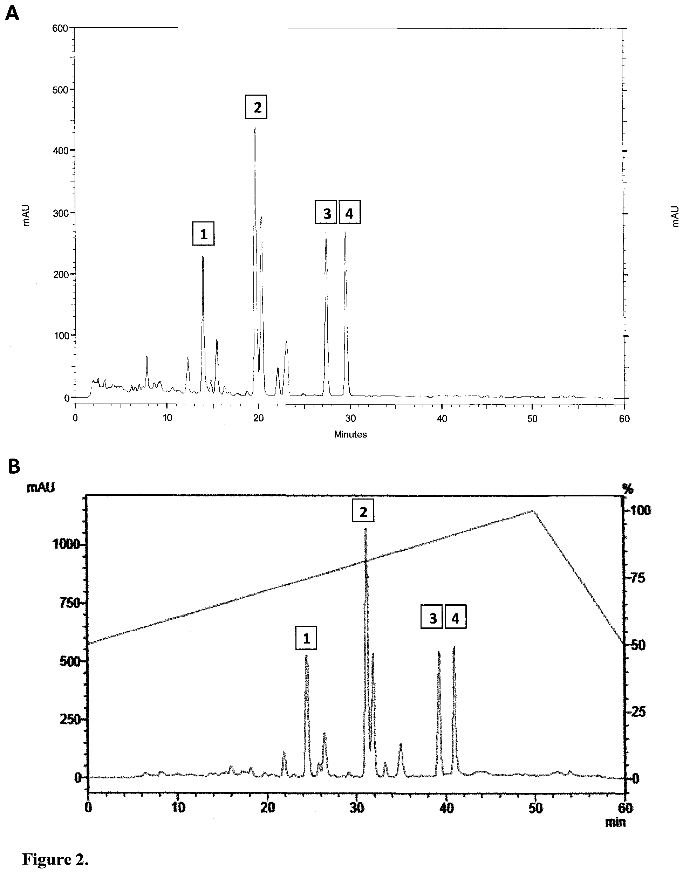

Figure 2 shows reverse-phase HPLC chromatograms of dan shen crude extract using the (A) analytical, and (B) semi-preparative columns. The calculated percentage yields from semi-preparative HPLC are listed in Table 1.

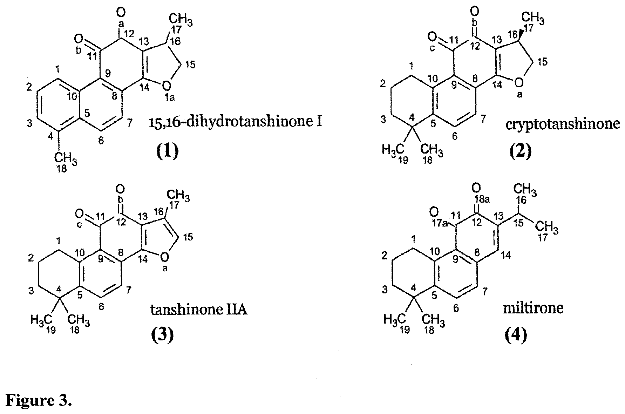

Figure 3 shows the molecular structures of the tanshinones (1) - (4) isolated from dan shen crude extract.

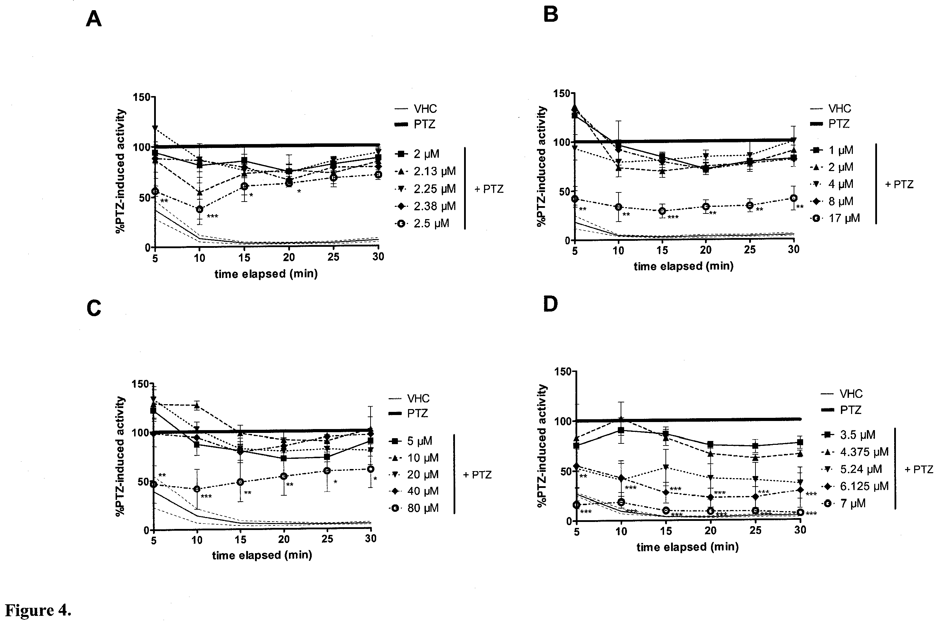

Figure 4 shows PTZ-normalized concentration response curves for (A) 15,16-dihydrotanshinone I, (B) cryptotanshinone, (C) tanshinone IIA and (D) miltirone. Results were normalized against PTZ controls (set at 100%). Analysis was done by two-way ANOVA, with P values < 0.05 (*), < 0.01 (**) and < 0.001 (***) indicated per time period.

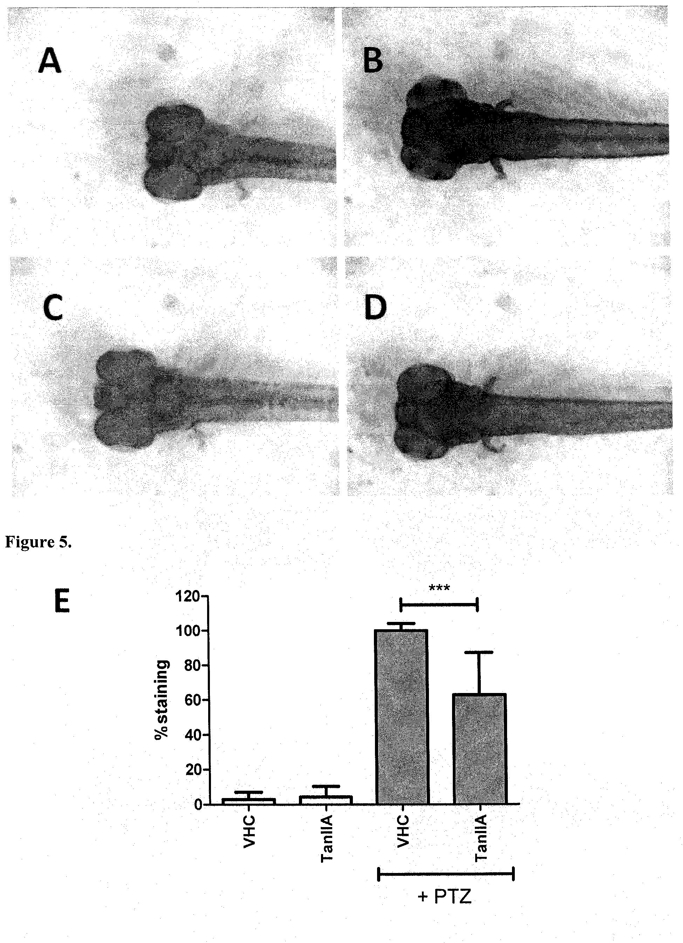

Figure 5 shows whole-mount *in situ* hybridization of 7-dpf zebrafish larvae to visualize the modulation of *c-fos* expression: (A) larva with no pre-treatment, vehicle only, unexposed to PTZ before fixation and staining; (B) no pre-treatment, vehicle only, then exposed to PTZ for 30 minutes before fixation and staining; (C) larva pre-treated with 80 µM tanshinone IIA for 18 hours, unexposed to PTZ before fixation and staining; and (D) larva pre-treated with 80 µM tanshinone IIA before PTZ exposure, fixation and staining. Fig. 5E shows results of quantitative analysis of WISH staining of 7-dpf zebrafish larvae to visualize the modulation of c-fos expression by vehicle and 80 µM tanshinone IIA (TanIIA) with (grey bars) or without (white bars) additional exposure to PTZ. Student's t-test was performed to determine statistical significance with P values < 0.05 (*), < 0.01 (**) and < 0.001 (***).

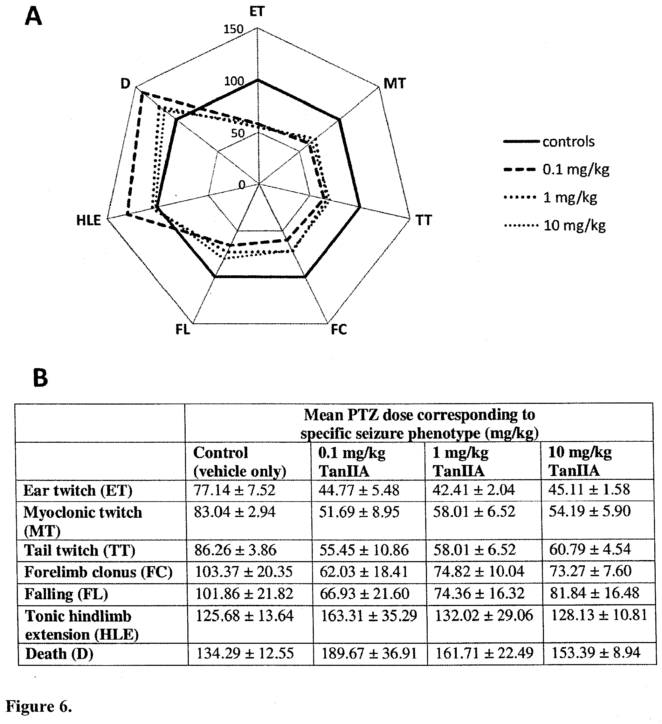

Figure 6 shows (A) radar graphs of PTZ-i.v. infused mice treated with tanshinone IIA. (n = 6 mice per treatment group). Results for each group were normalized against the controls (set at 100%). Mice were scored according to the PTZ dose used per seizure progression: ear twitch (ET), tail twitch (TT), myoclonic twitch (MT), forelimb clonus (FC), falling (F), tonic hindlimb extension (HLE) and death (D). Standard deviations were not included in the graph, but are listed in the corresponding table (B) with the mean PTZ doses for each treatment group. Statistical analysis was done using Student's t-test, with no significant difference determined for any treatment group as compared with controls.

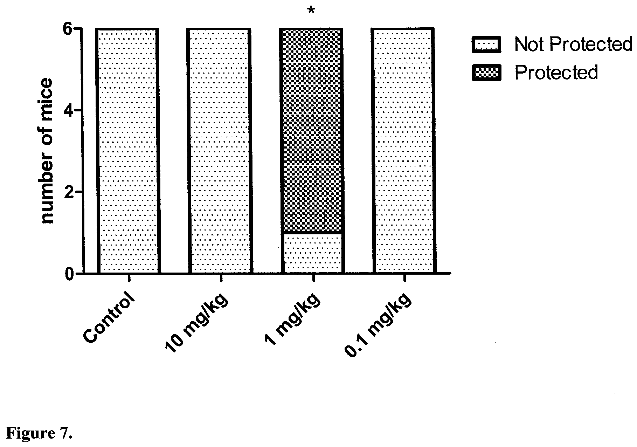

Figure 7 shows bar graphs of 6-Hz psychomotor seizure assay on male NMRI mice treated with tanshinone IIA. (n = 6 mice per treatment group). Analysis done using Fischer's exact test, at 95% confidence level.

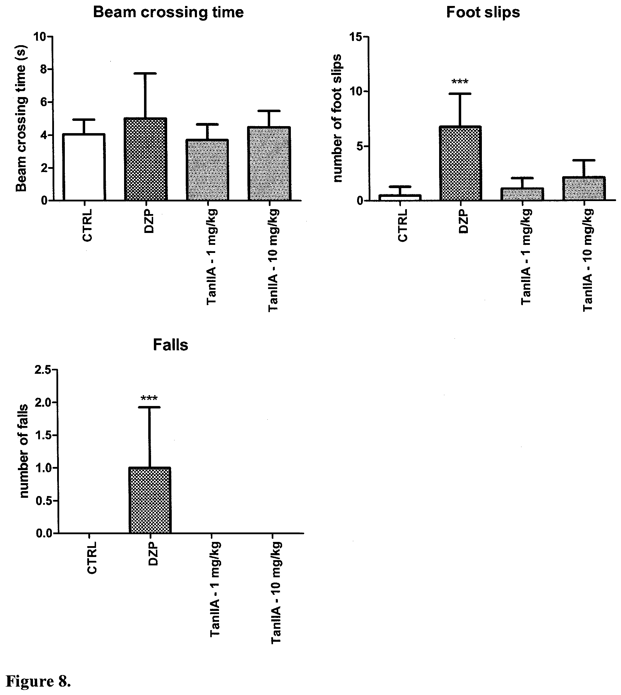

Figure 8 shows scores for beam-walking assay of male NMRI mice treated with 1 and 10 mg/kg tanshinone IIA (n = 6 mice per treatment group). Statistical analysis was done using one-way ANOVA with Dunnett's test, with P values < 0.05 (*), < 0.01 (**) and < 0.001 (***) indicated per treatment group.

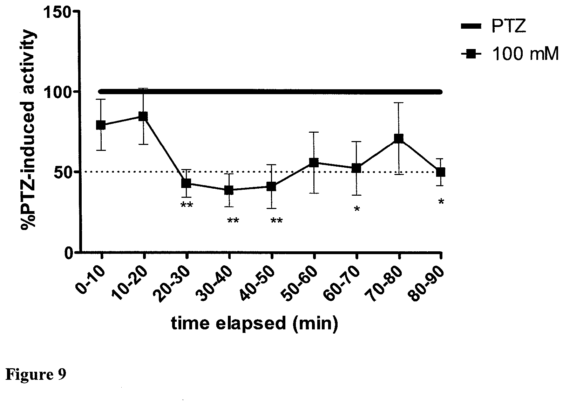

Figure 9 shows PTZ-normalized concentration response curves for intravenous injection of control (VHC) and tanshinone IIA (tan IIA) in 3-dpf zebrafish larvae that were subsequently exposed to 20 mM PTZ and tracked for 90 minutes. Results were normalized against PTZ controls (set at 100%). Analysis was done by two-way ANOVA, with P values < 0.05 (*), < 0.01 (**) and < 0.001 (***) indicated per time period.

### DETAILED DESCRIPTION

The inventions is defined by the appended claims.

The present disclosure relates to *Salvia miltiorrhiza,* extracts thereof, and compounds therefrom, including tanshinones, for use as anticonvulsant agents in treating neurological disorders which are characterized by seizures, such as epilepsy. Accordingly, anticonvulsant agents may be effective in reducing seizures, convulsions, and/or other involuntary changes in body movement or function. Anticonvulsant agents may also be known as antiepileptic agents, AEDs, anti-seizure agents, and/or seizure medications.

### Salvia miltiorrhiza and tanshinone compounds

*Salvia miltiorrhiza,* also known as red sage, Chinese sage, tan shen, or dan shen, is a plant from the genus *Salvia* and is used widely in traditional Chinese medicine. In China, a variety of ailments, such as chronic renal failure, coronary heart disease, and cerebrovascular disorders are treated with the root of S. *miltiorrhiza,* either alone or in combination with extracts from other plants. Additional studies have suggested that S. *miltiorrhiza* may also be useful for reduce blood clotting, liver fibrosis, growth of cancerous cells, HIV replication, and cell toxicity resulting from free radicals, among other uses.

The composition of a S. *miltiorrhiza* preparation depends on the method by which it is prepared, and thus the efficacy of each preparation must be determined. Any part of the S. *miltiorrhiza* plant may be used to prepare a composition. For example, the root of the plant may be used to prepare a composition. The whole root may be used, and/or parts of the root including but not limited to the primary root, lateral root, root hairs, root tip, and/or root cap may be used to prepare the composition. The non-root portion of the plant may also be used; i.e., leaves, stems, shoots, seeds, buds, flowers, and/or fruit may be used to prepare a composition.

One aspect of the present disclosure relates to an acetone extract of S. *miltiorrhiza* for use as an anticonvulsant in treating disorders of the central nervous system. In some aspects, the acetone extract is prepared from the root of S. *miltiorrhiza.* An acetone extract may be prepared according to the methods described herein. For example, dan shen root in the form of dried root powder may be dispersed in acetone via ultrasonication, with the resulting solution filtered and reduced to dryness. The resulting residue (DSH) may be dissolved in spectroscopic-grade dimethyl sulfoxide (DMSO) or dissolved in 1:1 DMSO:polyethylene glycol-200 (DMSO:PEG-200).

In some embodiments, the acetone extract comprises tanshinones. Tanshinones are cyclic compounds with a phenanthrene skeleton, and are phrenanthrenequinones. Exemplary tanshinones are represented by the following structures:

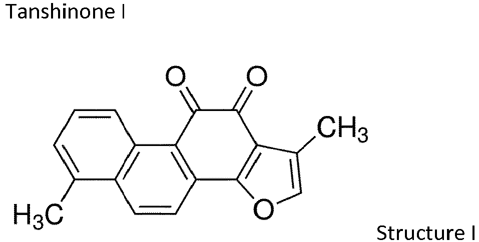

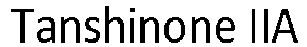

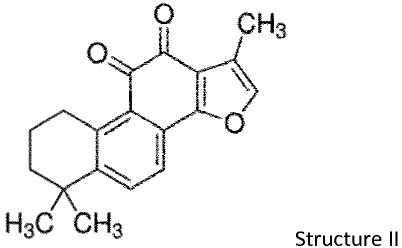

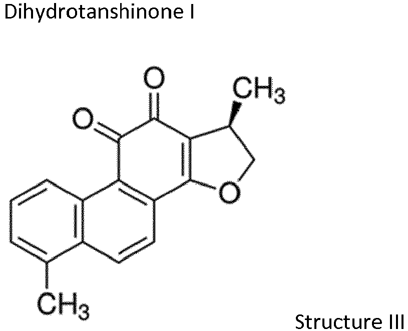

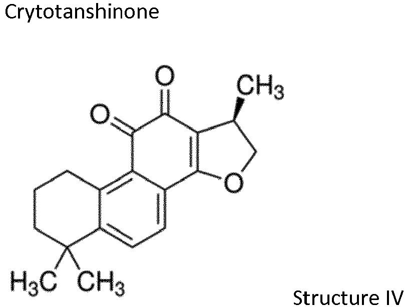

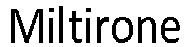

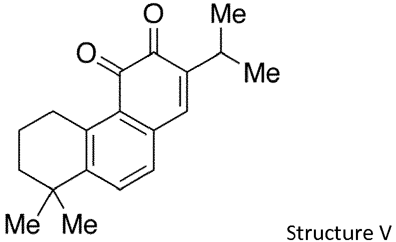

In some embodiments, the acetone extract comprises tanshinones. In some embodiments, the acetone extract comprises tanshinone IIA, cryptotanshinone, miltirone, and dihydrotanshinone I. The acetone extract may comprise at least one of tanshinone I (synonmyms: tanshinon I, Tanshinone A, Tanshinquinone I); tanshionone IIA (synonyms: 1,6,6-Trimethyl-6,7,8,9-tetrahydrophenanthro[1,2-b]furan-10,11-dione, Dan Shen ketone); cryptotanshionone (synonyms: 1,2,6,7,8,9-hexahydro-1,6,6-trimethyl- (R)-phenanthro(1,2-b)furan-10,11-dione, Cryptotanshinon, Tanshinone c); miltirone (synonyms: 5,6,7,8-Tetrahydro-8,8-dimethyl-2-(1-methylethyl)-3,4-phenanthrenedione; 5,6,7,8-Tetrahydro-2-isopropyl-8,8-dimethyl-3,4-phenanthrenedione; Miltiron; NSC 639662; Rosmariquinone); and dihydrotanshinone I (synonyms: (-)-Dihydrotanshinone I, 15,16-Dihydrotanshinone I, Dihydrotanshinone I). The acetone extract may comprise the exemplary tanshinones represented by at least one of structures I-V.

Another aspect of the present disclosure relates to a mixture of tanshinones for use in treating disorders of the central nervous system. In some embodiments, the mixture comprises at least two of tanshinone IIA, dihydrotanshinone I, cryptotanshinone and miltirone. In some embodiments, the mixture comprises tanshinone IIA and at least one of dihydrotanshinone I, cryptotanshinone and miltirone. In some embodiments, the mixture comprises at least two of tanshinone I, tanshinone IIA, dihydrotanshinone I, cryptotanshinone and miltirone. In some embodiments, the mixture comprises tanshinone IIA and at least one of tanshinone I, dihydrotanshinone I, cryptotanshinone and miltirone.

A further aspect of the present disclosure relates to tanshinone IIA for use as an anticonvulsant in treating disorders of the central nervous system. In some embodiments, the disorder of the central nervous system is epilepsy. Epilepsy may be a form of pharmacoresistant epilepsy and/or a form of genetic epilepsy. In certain aspects, the disorder of the central nervous system is characterized by involuntary movements. The involuntary movements may be associated with epilepsy, or may result from disorders such as trigeminal neuralgia and muscle spasms.

Still another embodiment of the present disclosure relates to an acetone extract of S. *miltiorrhiza,* a mixture of at least two tanshinones, and/or tanshionone IIA for use in treating epilepsy. In some embodiments, epilepsy is characterized by convulsions. In certain embodiments, epilepsy is not characterized by convulsions.

### Epilepsy and convulsive disorders

The term epilepsy describes a diverse set of neurological disorders which may be characterized by seizures, convulsions, and/or other involuntary changes in body movement or function. Approximately 65 million people worldwide are estimated to suffer from epilepsy, but effective treatment options are limited. To date, almost all currently available anti-epiletic drugs (AEDs) target neuronal receptors such as GABA receptors and sodium, glutamate, or calcium channels, in order to reduce neuronal excitability. However, traditional AEDs based on these targets have failed to control seizures in about 25-30% of all epilepsy patients, and further, some patients who initially respond to AEDs later experience drug-resistant (pharmacoresistant) seizures.

Epileptic seizures may result from any abnormal, excessive, or hypersynchronous neuronal activity in the brain. In some embodiments, epileptic seizures which require treatment with anticonvulsants are caused by infection, stroke, trauma, fever, tumors, drug use, damage to the blood-brain barrier, and/or neurodegenerative disease. In certain embodiments, epileptic seizures are triggered by emotional state, by response to light and/or sound, sleep, sleep deprivation, hormones, metabolic disorders, and/or congenital defects. Epileptic seizures for which the anticonvulsants disclosed herein provide treatment may be classified as partial seizures, such as simple partial seizures and/or complex partial seizures, or they may be classified as generalized seizures, such as absence seizures, myoclonic seizures, clonic siezures, tonic seizures, tonic-clonic seizures, and/or atonic seizures, or a mixed seizure. The seizures may be symptoms of temporal lobe epilepsy, whose features may include epileptic foci in the limbic system, an initial precipitating injury, a latent period, and the presence of hippocampal sclerosis leading to reorganization of neuronal networks.

The anticonvulsants described herein, such as extracts from S. *miltiorrhiza,* mixtures of tanshinones, and/or tanshinone IIA, may also provide treatment for therapy-resistant forms of seizure. Notably, the 6-Hz psychomotor seizure model of partial epilepsy has been used as a model therapy-resistant forms of seizures, including limbic seizures.

Patients suffering from epileptic seizures may be infants aged 0-6 months, 6-12 months, 12-18 months, 18-24 months. In certain aspects, patients suffering from epileptic seizures are individuals aged 65-70, 75-80, 85-90, 95-100, 100-105, and older. Patients may also be children aged 2-12, adolescents aged 13-19, or adults aged 20-64.

Anticonvulsants may be used for the treatment of epileptic seizures, including treatment of symptoms associated with epileptic seizures and/or epilepsy. Anticonvulsants may also be used to treat epileptic seizures that result from central nervous system disorders such as cerebrovascular diseases and/or neurodegenerative diseases. One goal of an anticonvulsant agent (i.e., an "anticonvulsant") is to suppress the rapid and excessive firing of neurons that start a seizure. Another goal of an anticonvulsant is to prevent the spread of the seizure within the brain and offer protection against possible excitotoxic effects, that may result in brain damage. Anticonvulsants may also be referred to as antiseizure drugs or anti-epileptic drugs (AEDs). In epilepsy, an area of the brain and/or nervous system is typically hyper-irritable. Antiepileptic drugs function to help reduce this area of irritability and thus prevent epileptic seizures.

Epilepsy may be a form of pharmacoresistant epilepsy and/or a form of genetic epilepsy. In some aspects, epilepsy is not characterized by convulsions or seizures, but reflects abnormal, excessive, or hypersynchronous neuronal activity in the brain.

### EXAMPLES

Having provided a general disclosure, the following examples help to illustrate the general disclosure. These specific examples are included merely to illustrate certain aspects and embodiments of the disclosure, and they are not intended to be limiting in any respect. Certain general principles described in the examples, however, may be generally applicable to other aspects or embodiments of the disclosure.

### Example 1. Activity of crude acetone extract of dan shen in zebrafish-PTZ assay

Zebrafish larvae were exposed to different concentrations of the acetone crude extract of dan shen for 1 hour (for 7-dpf larvae) and 18 hours (for 6-dpf larvae) before PTZ treatment and activity tracking. Dan shen crude extract reduced PTZ-induced activity in larvae after 1-hr exposure time at MTC (5 µg/mL), but not after 18-hours of exposure (Figure 1). Beyond the MTC, larvae displayed bradycardia, loss of posture and delayed touch response after 3 hours exposure, followed by death after 18 hours.

### Example 2. Isolation and identification of tanshinones present in crude acetone extract of dan shen, and evaluation in zebrafish

The crude acetone extract of dan shen was subjected to analytical HPLC analysis, which revealed the presence of eleven peaks (Figure 2). In order to differentiate the peaks in terms of their intrinsic activity aside from approximate abundance, these were initially dissolved in equivolume amounts (10 µL) of DMSO before exposing to 7-dpf larvae in 0.3x Danieau's solution (1% final DMSO concentration) for 18 hours before subsequent exposure to PTZ; peaks which showed signs of toxicity in larvae after the pre-exposure period were titered from half to the tenth of their original unknown concentrations. Four peaks were found to be active in this manner (data not shown) and became the focus of semi-preparative isolation for struc-ture elucidation via NMR spectroscopy.

One- and two-dimensional NMR analyses of the isolated peaks showed that they are structurally related to each other as tanshinones (Table 1). Active peaks were identified as 15,16-dihydrotanshinone I **(1),** cryptotanshinone **(2),** tanshinone IIA **(3)** and miltirone **(4)** (Figure 3).

Concentration-response curves were created for the active tanshinones. The MTC values for **1, 2, 3** and **4** were 2.5, 17, >80 (at maximum solubility) and 7 µM respectively, with greatest reduction in PTZ-induced activity in 7-dpf larvae at these concentrations (Figure 3A-D). 15,16-Dihydrotanshinone I **(1)** at its active concentration, however, did not display consistent reduction of PTZ-induced activity over the course of 30 minutes compared to the other tan-shinones (Figure 4A). Cryptotanshinone **(2)** displayed significant reduction of PTZ-related activity only at its MTC (Figure 4B), unlike miltirone **(4)** with its effective concentration lower than its MTC (Figure 4D). Tanshinone IIA (3), at its MTC value of 80 µM, also dis-played significant reduction of PTZ-induced activity throughout the duration of the assay (Figure 4C). As noted in earlier literature, tanshinone IIA has been identified as a predominant bioactive constituent of dan shen and developed as a prescription drug for cardio- and cerebrovascular disorders, therefore we chose to focus on this specific compound for further experiments.

### Example 3. Intravenous micro-injection of tanshinone IIA in zebrafish larvae

To compensate for tanshinone IIA's poor solubility in larval medium, additional locomotor recordings were made on intravenously (i.v.)-injected 3-dpf zebrafish larvae with either vehicle (1:1 DMSO:0.3x Danieau's solution) or 100 mM tanshinone IIA solutions. I.v.-injected tanshinone IIA significantly reduced PTZ-induced movement in 3-dpf larvae (Figure 9). Taking into account the bolus volume (1 nL), concentration (100 mM) and average weight of a 3-dpf larva (0.25 mg), the estimated calculated dose is 59 mg/kg larva (Figure 9).

### Example 4. Effect on c-fos expression of tanshinone IIA in zebrafish larvae

Previous studies have shown the robustness of *in situ* hybridization analysis of *c-fos* transcription in zebrafish larval central nervous system as a high-throughput indicator of the neural response to chemoconvulsant treatment in lieu of electroencephalographic (EEG) recordings [6,12]. Confirmation of neuroprotective activity of tanshinone IIA was done through whole-mount *in situ* hybridization (WISH) of 6/7-dpf larvae which were pre-exposed to the samples for 18 hours before subsequent exposure to PTZ. Overexpression of the oncogene *c-fos* in the brain was scored for sham-, vehicle- and sample-treated larvae, as the extent of *c-fos* overexpression has been linked to severity of seizures and neuronal insults in the brain [12]. Larvae exposed to the tanshinone IIA (80 µM) for 18 hours before PTZ treatment show reduction of *c-fos* expression (Figure 5) compared to larvae pre-exposed to vehicle, with significant reduction of staining at the hindbrain. Quantitative analysis confirmed the reduction of *c-fos* staining in the brain by as much as one-third (Fig. 5e).

### Example 5. Evaluation of tanshinone IIA in rodent models

We explored the potential activity of the dan shen crude extract in two standard rodent seizure models-the timed i.v. PTZ infusion test, and the 6-Hz corneal stimulation in mice. Tanshinone IIA at 10, 1 and 0.1 mg/kg injected i.v. into mice displayed a more complex pattern upon continuous i.v.-PTZ infusion (Figure 6), wherein reduction of seizure threshold was observed at the initial stage of the PTZ infusion, but coupled with a subsequent increase in threshold at the latter stages (Table 2). In the 6-Hz psychomotor seizure assay, only 1 mg/kg tanshinone IIA was successful in protecting 5 out of 6 mice (Figure 7).

To complement the rodent seizure assays for tanshinone IIA, we wanted to determine if the selected dose-ranges could possibly contribute to neurological deficits by themselves. NMRI mice were injected i.p. with 10 and 1 mg/kg of the compound and were subjected to the beam-walking assay. Both groups of mice performed as well as mice injected with vehicle only, which fared better than those injected with diazepam (1 mg/kg) that resulted in an increase in the number of foot slips and falls (Figure 8).

### MATERIALS & METHODS

Plant materials and reagents. Dried dan shen root powder was obtained from the Department of Chemistry, National Tsinghua University. Tanshinone IIA and dihydrotanshinone I standards were sourced from IMAM International Pharmaceuticals, Ltd. (Tianjin, China).

Crude extract preparation. Five grams of dried dan shen root powder was dispersed in 50-mL acetone via ultrasonication (Bransonic 5510E-MT: Danbury 06813, USA), with the resulting solution filtered and reduced to dryness (60 mg). The resulting residue (DSH) was dissolved in spectroscopic-grade dimethyl sulfoxide (DMSO: Acros Organics; Geel, Belgium) as 20 mg/mL stock solution. For rodent assays, the residue was dissolved in 1:1 DMSO:polyethylene glycol-200 (DMSO:PEG-200) as 100 mg/mL stock solution. All stock solutions were stored at -20°C.

High-performance liquid chromatography (HPLC). Approximately 200 µg of the crude extract was dissolved in 20 µL HPLC-grade acetonitrile (ACN: Acros Organics; Geel, Belgium) and was injected in a Hitachi EZChrom Elite HPLC system with a Luna C-18(2) reversed-phase silica analytical column (Phenomenex: Torrance, CA, USA), quaternary solvent pump and a diode array detector. A gradient solvent system (0-40 min, 50% ACN/water-100% ACN; 41-60 mins, 100% ACN-50% ACN/water) at 0.8 mL/min was used to resolve the components in the extract. For semi-preparative work, 10 mg of the extract was injected in a Gilson 712 HPLC system with a Waters 2487 dual-wavelength detector (Waters USA: Milford, MA, USA) and Sun Fire Prep C-18 reversed-phase semi-preparative column (10x250 mm, 5 µm; Waters USA: Milford, MA, USA). A similar gradient solvent system at 2.5 mL/min was used in this scheme to isolate fractions of the components. The isolated fractions were subject to rotary evaporation and purged with dry N2 gas to obtain residues for further analyses.

Nuclear magnetic resonance spectroscopy (NMR). The residues were dissolved in 2.5 mL deuterated DMSO (Sigma-Aldrich: St. Louis, MO, USA) in 5-mm i.d. glass NMR tubes, and run in Bruker Avance 600-MHz NMR spectrometer. Data and structure analyses for proton and carbon signals were done using ACDLabs NMR Processor Academic Edition 12 (ACDLabs: Cambridge, MA, USA).

Zebrafish. Adult zebrafish (AB strain) were reared at 28°C on a 14/10-hour light/dark cycle according to standard aquaculture conditions. Eggs were collected following natural spaw-ning, sorted and raised in 0.3x Danieau's solution under constant light conditions in an incu-bator set at 28°C until seven days post-fertilization (7-dpf).

Mice. Male 10- to 12 week old C57BI/6 and NMRI mice were kept in 12/12-hour light/dark cycle and were provided with standard rodent diet ad libitum. All aspects of animal experiment and husbandry were carried out in compliance with the National and European Regulations and were approved by the Animal Care and Use Committee of the University of Leuven.

Automated larval zebrafish-PTZ assay. One 7-dpf zebrafish larva was placed per well in a 96-well plate format. Excess larval medium was removed and replaced with 100 µL of either control or sample solutions. The prepared plate was placed in a dark box inside an incubator set at 28°C for 1 or 18 hours, then inspected per well for signs of toxicity (e.g., irregular heart-rate, loss of posture, edema, necrosis, delayed startle or touch response). The maximum tolerated concentration (MTC) was designated to the highest sample concentration that did not elicit any signs of toxicity in 6/7-dpf larvae after 18 hours of exposure. Upon addition of 100 µL of 40 mM PTZ per well, the plate was positioned in the zebrafish tracking box, equilibrated for 5 minutes before recording for 30 minutes. Tracking data was exported into Excel format and processed as such before statistical analysis via GraphPad™ Prism v.5 for Windows. Each tracking data set were normalized against the PTZ-only control values (set at 100%) within each set, with each subsequent replicate set pooled before two-way ANOVA with Bonferroni post hoc analysis.

Intravenous microinjection of tanshinone IIA into 3-dpf zebrafish larvae. In order to overcome potential issues with tanshinone IIA's solubility in the larval medium, i.v. micro-injections were performed. Three-dpf zebrafish larvae were anesthetisized with tricaine before securing them on a grooved agarose mold in supine position. One nanoliter of tanshinone IIA (100 mM in 50% DMSO) was injected into the circulation through the Duct of Cuvier (future common cardinal vein; CCV) [18] using a pulled-glass capillary needle. Injected larvae were left to recover in tricaine-free larval medium in 96-well plates for 30 minutes before exposure to 20 mM PTZ for automated locomotor assays, with corresponding sets of sham-injected and uninjected larvae for controls.

Whole mount *in situ* hybridization (WISH) of 7-dpf zebrafish larvae for brain *c-fos* expression. This assay was performed to assess the extent of seizure suppression in zebrafish brains, as increased *c-fos* expression serves as a well-documented indicator for seizure onset in both fish and mammalian models [6,12]. At least 30 6/7-dpf larvae were exposed to 1 mL vehicle and sample solutions 18 hours before addition of 1 mL 40 mM PTZ for 3§0 minutes. The treated larvae were fixed in 4% paraformaldehyde overnight, then exposed to 10 µg/mL proteinase K before hybridization with digoxigenin-labelled c-fos RNA probe, according to standard procedures [6,12,13].

Timed PTZ-infusion mouse assay. Twenty-four C57BI/6J mice were divided into control and treatment groups of 6 mice each. Mice were restrained, and their lateral veins were catheterized with 1-cm 29-gauge needle attached to polyethylene tubing and secured with surgical tape. One-hundred microliters of one of the following solutions (vehicle-1:1 DMSO:PEG200, different concentrations of dan shen crude extract and tanshinone IIA) were i.v.-infused into mice for two (2) minutes at 50µL/min using an automated i.v. injector pump (World Precision Instruments ALADOIN-1000 11VDC, 0.75Å), then incubated for ten (10) minutes before shifting to a continuous infusion of 7.5 mg/mL PTZ (0.9% saline water) at 150 µL/min. Mice were scored according to the time-latencies of seizure progression: ear twitch (ET), tail twitch (TT), myoclonic twitch (MT), forelimb clonus (FC), falling (F), tonic hindlimb extension (HLE) and death (D). All surviving mice were euthanized immediately at the end of infusion.

6-Hz psychomotor seizure assay in mice. NMRI mice of approximately 30-35 g were grouped by six per cage. Each mouse was injected i.p. with one of the following solutions-vehicle (1:1 DMSO:PEG200), different concentrations of dan shen crude extract and tanshinone IIA-30 minutes before the procedure. Seizures are induced via corneal stimulation using (Ugo-Basil device). Prior to the placement of corneal electrodes, a drop of 0.5% xylocaine is applied to the eyes of the animal. Animals are restrained manually and released immediately in a cage of plexi glass following the stimulation. Then the animal is observed for characteristic signs such as stun, forelimb clonus, twitching of vibrissae, and Straub tail for at least 45 seconds. Protection is defined as the absence of a seizure within the expected time frame.

Beam-walking test. NMRI mice of approximately 30-35 g were grouped by six per cage and were trained three times to walk across an 80-cm wooden beam from an open platform. Each mouse was injected i.p. with one of the following solutions-vehicle, tanshinone IIA (10 and 1 mg/kg) and diazepam (1 mg/kg)-30 minutes before the actual experiment. The duration of the crossings, number of foot slips and falls were recorded per mouse for three trials.

### TABLES

**Table 1. Fractions isolated using semi-preparative reverse-phase HPLC analysis of dan shen crude extract (DSH, 130.6 mg), with yields expressed as percentage (%) of the extract or residue.**

| **HPLC peak label** | **Weight of peak residue (mg)** | **% in DSH crude residue** | **Peak identity (via NMR)** |
|---|---|---|---|
| 1 | 6.13 | 4.7 | 15,16-dihydrotanshinone I |
| 2 | 17.07 | 13.1 | cryptotanshinone |
| 3 | 10.12 | 7.7 | tanshinone IIA |
| 4 | 3.68 | 2.8 | miltirone |

**Table 2. Mean PTZ dose corresponding to specific seizure phenotype per treatment group correlated to the radar graph in Figure 9. Statistical analysis was done using one-way ANOVA with Dunnett's test, with significant differences (indicated by *, P < 0.001) seen for all tanshinone IIA treatment sets.**

| | **Mean PTZ dose corresponding to specific seizure phenotype (mg/kg)** | | | |
|---|---|---|---|---|
| | **Control (vehicle only)** | **0.1 mg/kg TanIIA** | **1 mg/kg TanIIA** | **10 mg/kg TanIIA** |
| **Ear twitch (ET)** | 77.1 ± 7.5 | *44.7 ± 5.4 | *42.4 ± 2.0 | *45.1 ± 1.5 |
| **Myoclonic twitch (MT)** | 83.0 ± 2.9 | *51.6 ± 8.9 | *58.0 ± 6.5 | *54.1 ± 5.9 |
| **Tail twitch** (TT) | 86.2 ± 3.8 | *55.4 ± 10.8 | *58.0 ± 6.5 | *60.7 ± 4.5 |
| **Forelimb clonus (FC)** | 103.3 ± 20.3 | *62.0 ± 18.4 | 74.8 ± 10.0 | 73.2 ± 7.6 |
| **Falling (FL)** | 101.8 ± 21.8 | *66.9 ± 21.6 | 74.3 ± 16.3 | 81.8 ± 16.4 |
| **Tonic hindlimb extension (HLE)** | 125.6 ± 13.6 | *163.3 ± 35.2 | 132.0 ± 29.0 | 128.1± 10.8 |
| **Death (D)** | 134.2 ± 12.5 | *189.6 ± 36.9 | *161.7 ± 22.4 | *153.3 ± 8.9 |

### EQUIVALENTS

While specific embodiments of the subject invention have been discussed, the above specification is illustrative and not restrictive. Many variations of the invention will become apparent to those skilled in the art upon review of this specification and the claims below. The full scope of the invention should be determined by reference to the claims, along with their full scope of equivalents, and the specification, along with such variations.

### REFERENCES

1. World Health Organization (2012) Epilepsy Fact Sheet No. 999. In: http://www.who.int/mediacentre/factsheets/fs999/en/index.html.
2. Saslis-Lagoudakis CH, Savolainen V, Williamson EM, Forest F, Wagstaff SJ, et al. (2012) Phylogenies reveal predictive power of traditional medicine in bioprospecting. Proc Natl Acad Sci U S A 109: 15835-15840.
3. Crawford AD, Esguerra CV, de Witte PA (2008) Fishing for drugs from nature: zebrafish as a technology platform for natural product discovery. Planta Med 74: 624-632.
4. Orellana-Paucar AM, Serruys AS, Afrikanova T, Maes J, De Borggraeve W, et al. (2012) Anticonvulsant activity of bisabolene sesquiterpenoids of Curcuma longa in zebrafish and mouse seizure models. Epilepsy Behav 24: 14-22.
5. Baraban SC (2007) Emerging epilepsy models: insights from mice, flies, worms and fish. Curr Opin Neurol 20: 164-168.
6. Baraban SC, Taylor MR, Castro PA, Baier H (2005) Pentylenetetrazole induced changes in zebrafish behavior, neural activity and c-fos expression. Neuroscience 131: 759-768.
7. Afrikanova T, Serruys AS, Buenafe OE, Clinckers R, Smolders I, et al. (2013) Validation of the zebrafish pentylenetetrazol seizure model: locomotor versus electrographic responses to antiepileptic drugs. PLoS One 8: e54166.
8. Berghmans S, Hunt J, Roach A, Goldsmith P (2007) Zebrafish offer the potential for a primary screen to identify a wide variety of potential anticonvulsants. Epilepsy Res 75: 18-28.
9. Sucher NJ (2006) Insights from molecular investigations of traditional Chinese herbal stroke medicines: implications for neuroprotective epilepsy therapy. Epilepsy Behav 8: 350-362.
10. Zhou L, Zuo Z, Chow MS (2005) Danshen: an overview of its chemistry, pharmacology, pharmacokinetics, and clinical use. J Clin Pharmacol 45: 1345-1359.
11. Sun X, Chan LN, Sucher NJ (2005) Magnesium as NMDA receptor blocker in the traditional Chinese medicine Danshen. Phytomedicine 12: 173-177.
12. Baxendale S, Holdsworth CJ, Meza Santoscoy PL, Harrison MR, Fox J, et al. (2012) Identification of compounds with anti-convulsant properties in a zebrafish model of epileptic seizures. Dis Model Mech 5: 773-784.
13. Thisse C, Thisse B (2008) High-resolution in situ hybridization to whole-mount zebrafish embryos. Nat Protoc 3: 59-69.
14. Wang X, Morris-Natschke SL, Lee KH (2007) New developments in the chemistry and biology of the bioactive constituents of Tanshen. Med Res Rev 27: 133-148.
15. Adams JD, Wang R, Yang J, Lien EJ (2006) Preclinical and clinical examinations of Salvia miltiorrhiza and its tanshinones in ischemic conditions. Chin Med 1: 3.
16. Kim SY, Moon TC, Chang HW, Son KH, Kang SS, et al. (2002) Effects of tanshinone I isolated from Salvia miltiorrhiza bunge on arachidonic acid metabolism and in vivo inflammatory responses. Phytother Res 16: 616-620.
17. Tang MK, Ren DC, Zhang JT, Du GH (2002) Effect of salvianolic acids from Radix Salviae miltiorrhizae on regional cerebral blood flow and platelet aggregation in rats. Phytomedicine 9: 405-409.
18. Xu S, Liu P (2013) Tanshinone II-A: new perspectives for old remedies. Expert Opin Ther Pat 23: 149-153.
19. Liu L, Zhang X, Wang L, Yang R, Cui L, et al. (2010) The neuroprotective effects of Tanshinone IIA are associated with induced nuclear translocation of TORC1 and upregulated expression of TORC1, pCREB and BDNF in the acute stage of ischemic stroke. Brain Res Bull 82: 228-233.
20. Tian XH, Wu JH (2013) Tanshinone derivatives: a patent review (January 2006 - September 2012). Expert Opin Ther Pat 23: 19-29.
21. Lee CM, Wong HN, Chui KY, Choang TF, Hon PM, et al. (1991) Miltirone, a central benzodiazepine receptor partial agonist from a Chinese medicinal herb Salvia miltiorrhiza. Neurosci Lett 127: 237-241.
22. Chang HM, Chui KY, Tan FW, Yang Y, Zhong ZP, et al. (1991) Structure-activity relationship of miltirone, an active central benzodiazepine receptor ligand isolated from Salvia miltiorrhiza Bunge (Danshen). J Med Chem 34: 1675-1692.
23. Calabrese EJ (2008) Hormesis and medicine. Br J Clin Pharmacol 66: 594-617.
24. Calabrese EJ (2008) Modulation of the epileptic seizure threshold: implications of biphasic dose responses. Crit Rev Toxicol 38: 543-556.
25. Hao H, Wang G, Cui N, Li J, Xie L, et al. (2006) Pharmacokinetics, absorption and tissue distribution of tanshinone IIA solid dispersion. Planta Med 72: 1311-1317.
26. Xu S, Little PJ, Lan T, Huang Y, Le K, et al. (2011) Tanshinone II-A attenuates and stabilizes atherosclerotic plaques in apolipoprotein-E knockout mice fed a high cholesterol diet. Arch Biochem Biophys 515: 72-79.
27. Adams JD, Wang R, Yang J, Lien EJ (2006) Preclinical and clinical examinations of Salvia miltiorrhiza and its tanshinones in ischemic conditions. Chin Med 1: 3.
28. Kim SY, Moon TC, Chang HW, Son KH, Kang SS, et al. (2002) Effects of tanshinone I isolated from Salvia miltiorrhiza bunge on arachidonic acid metabolism and in vivo inflammatory responses. Phytother Res 16: 616-620.
29. Tang MK, Ren DC, Zhang JT, Du GH (2002) Effect of salvianolic acids from Radix Salviae miltiorrhizae on regional cerebral blood flow and platelet aggregation in rats. Phytomedicine 9: 405-409.
30. Xu S, Liu P (2013) Tanshinone II-A: new perspectives for old remedies. Expert Opin Ther Pat 23: 149-153.
31. Liu L, Zhang X, Wang L, Yang R, Cui L, et al. (2010) The neuroprotective effects of Tanshinone IIA are associated with induced nuclear translocation of TORC1 and upregulated expression of TORC1, pCREB and BDNF in the acute stage of ischemic stroke. Brain Res Bull 82: 228-233.
32. Tian XH, Wu JH (2013) Tanshinone derivatives: a patent review (January 2006 - September 2012). Expert Opin Ther Pat 23: 19-29.
33. Wang WM, Zhang YQ, Wu YP, Fan ZY, Yang YX, Ye J (2013) Repaired abnormal perfusion foci in children with epilepsy trough traditional Chinese medicine compound danshen dripping pills assisted with valproic acid (110 case): To evaluate in a new treatment through interictcal SPECT and long-term V-EEG and imaging. Epilepsia 54: 30-340, P177
34. WO 02/12218 A1

## Claims

1. An acetone extract of *Salvia miltiorrhizha* for use as an anticonvulsant in treating epilepsy.

2. The acetone extract of *Salvia miltiorrhiza* for use according to claim 1, wherein the acetone extract comprises tanshinones.

3. The acetone extract of *Salvia miltiorrhiza* for use according to claim 2, wherein the tanshinones comprise dihydrotanshinone I, cryptotanshinone, tanshinone IIA, and miltirone.

4. A mixture of tanshinones for use as an anticonvulsant in treating epilepsy.

5. The mixture of tanshinones for use according to claim 4, wherein the mixture comprises at least two of dihydrotanshinone I, cryptotanshinone, tanshinone IIA, and miltirone.

6. The mixture of tanshinones for use according to claim 5, wherein the mixture comprises tanshinone IIA and at least one of dihydrotanshinone I, cryptotanshinone, and miltirone.

7. Tanshinone IIA for use as an anticonvulsant in treating epilepsy.

## Patentansprüche

1. Ein Aceton-Extrakt von *Salvia miltiorrhizha* zur Verwendung als Antikonvulsivum bei der Behandlung von Epilepsie.

2. Das Aceton-Extrakt von *Salvia miltiorrhiza* zur Verwendung nach Anspruch 1, wobei der Aceton-Extrakt Tanshinone umfasst.

3. Das Aceton-Extrakt von *Salvia miltiorrhiza* zur Verwendung nach Anspruch 2, wobei die Tanshinone Dihydrotanshinon I, Cryptotanshinon, Tanshinon IIA und Miltiron umfassen.

4. Ein Gemisch von Tanshinonen zur Verwendung als Antikonvulsivum bei der Behandlung von Epilepsie.

5. Das Gemisch von Tanshinonen zur Verwendung nach Anspruch 4, wobei das Gemisch mindestens zwei von Dihydrotanshinon I, Cryptotanshinon, Tanshinon IIA und Miltiron umfasst.

6. Gemisch von Tanshinonen zur Verwendung nach Anspruch 5, wobei das Gemisch Tanshinon IIA und mindestens eines von Dihydrotanshinon I, Cryptotanshinon und Miltiron umfasst.

7. Tanshinon IIA zur Verwendung als Antikonvulsivum bei der Behandlung von Epilepsie.

## Revendications

1. Extrait acétonique de *Sal₇la miltiorrhizha* pour une utilisation en tant qu'anticonvulsivant dans le traitement de l'épilepsie.

2. Extrait acétonique de *Sal₇la miltiorrhizha* pour une utilisation selon la revendication 1, dans lequel l'extrait acétonique comprend des tanshinones.

3. Extrait acétonique de *Sal₇la miltiorrhizha* pour une utilisation selon la revendication 2, dans lequel les tanshinones comprennent de la dihydrotanshinone I, de la cryptotanshinone, de la tanshinone IIA et de la miltirone.

4. Mélange de tanshinones pour une utilisation en tant qu'anticonvulsivant dans le traitement de l'épilepsie.

5. Mélange de tanshinones pour une utilisation selon la revendication 4, dans lequel le mélange comprend au moins deux éléments parmi la dihydrotanshinone I, la cryptotanshinone, la tanshinone IIA et la miltirone.

6. Mélange de tanshinones pour une utilisation selon la revendication 5, dans lequel le mélange comprend de la tanshinone IIA et au moins un élément parmi la dihydrotanshinone I, la cryptotanshinone et la miltirone.

7. Tanshinone IIA pour une utilisation en tant qu'anticonvulsivant dans le traitement de l'épilepsie.
